# EUROPÄISCHE PATENTSCHRIFT

(11) **EP 0 436 855 B1**
(45) Veröffentlichungstag und Bekanntmachung des Hinweises auf die Patenterteilung: **01.06.1994**
(21) Anmeldenummer: 90123829.5
(22) Anmeldetag: 11.12.1990
(51) Int. Cl.: A61M 1/16, A61L 2/00

(54) **Verfahren und Anordnung zum Desinfizieren eines Dialysierflüssigkeitskreislaufes**
Method and means for desinfection of a dialysate circuit
Procédé et dispositif pour la désinfection d'un circuit de dialysat

(30) Priorität: 13.12.1989 DE 3941103
(43) Veröffentlichungstag der Anmeldung: 17.07.1991
(73) Patentinhaber: MEDICAL SUPPORT GMBH, D-63110 Rodgau (DE)
(72) Erfinder:
(74) Vertreter: Flosdorff, Jürgen, Dr.

(56) Entgegenhaltungen:
- FR-A- 2 073 337

## Beschreibung

Die Erfindung betrifft ein Desinfektionsverfahren eines Dialysierflüssigkeitskreislaufes, bestehend aus einer Dialysemaschine mit einer ersten Stichleitung, die an ein Zulaufhauptventil einer Frischwasserzulaufleitung angeschlossen wird, und einer zweiten Stichleitung, die an ein Ablaufhauptventil einer Wasserablaufleitung angeschlossen wird, wobei dem Zulaufhauptventil ein Zulaufvorventil und dem Ablaufhauptventil ein Ablaufvorventil jeweils an der von dem Dialysegerät abgewandten Seite vorgeschaltet ist und eine mit einem Querventil versehene Querverbindungsleitung die Leitungsabschnitte zwischen den Haupt- und Vorventilen verbindet. Die Erfindung betrifft ferner eine Anordnung zum Desinfizieren eines Dialysierflüssigkeitskreislaufes, und zwar ein Gerät zur Durchführung von Hygienemaßnahmen an einem Dialysegerät, eine Wasserversorgungseinrichtung für ein Dialysegerät mit einer Frischwasserzulaufleitung mit einem Zulaufventil, an das eine erste Stichleitung des Dialysegerätes anschließbar ist, und mit einer Wasserablaufleitung mit einem Ablaufventil, an das eine zweite Stichleitung des Dialysegerätes anschließbar ist, sowie eine Zuführeinrichtung zum Zuführen von zwei Flüssigkeiten, nämlich einem Desinfektionsmittel in einer vorbestimmten Dosis und Wasser bei dem Desinfektionsverfahren.

In einem Dialysierflüssigkeitskreislauf gibt es eine Vielzahl möglicher Kontaminationsquellen, von denen insbesondere die Stichleitungen des Dialysegerätes mit Ansaugstücken und Verschraubungen, das Osmosewasser sowie die Vermischung frischer Dialysierflüssigkeit mit verbrauchter Dialysierflüssigkeit zu nennen sind. Um die heute in der Dialyse bekannten Bakterien und Viren abzutöten, sind bisher drei Desinfektionsverfahren bekannt, die in die Wirkungsbereiche Heißreinigung, Autoklavieren und chemische Desinfektion eingegliedert sind.

Allen diesen bekannten Verfahren "Heißreinigung , Autoklavieren und chemische Desinfektion" liegt lediglich die partielle Maschinendesinfektion zugrunde. Dies bedeutet, daß zwar das Dialysegerät bzw. die Dialysemaschine desinfiziert wird, nicht jedoch deren Verteilungssystem, so daß unmittelbar bei Beginn der Dialyse die desinfizierte Maschine mittels unsteriler Stichleitungen und Ansaugstücke wieder kontaminiert werden kann.

Weitere Nachteile der bekannten Verfahren bestehen in einer hohen thermischen Materialbelastung während der Autoklavierung, wobei hierin eine Ursache für häufig auftretende vorzeitige Komponentenausfälle im Hydraulikkreislauf liegen dürfte. Ferner kann wegen der automatischen Zwangsspülung nach einer chemischen Desinfektion eine Kontamination über das Osmosewasser verursacht werden. Nachteilig ist ferner der bisher erforderliche beträchtliche Energieaufwand im Zusammenhang mit der Durchlauferhitzung bei der Heißreinigung sowie der große Zeitaufwand von Desinfektionsmaßnahmen zwischen zwei Dialysebehandlungen.

Aus der FR-A 2973337 ist eine sterilisierbare Vorrichtung zur Herstellung einer Dialysierflüssigkeit bekannt, die eine Einlaßleitung zur Versorgung der Mischeinrichtung mit Trägerflüssigkeit, eine Einlaßleitung für ein Dialysierflüssigkeitskonzentrat, eine Auslaßleitung von der Mischeinrichtung zum Dialysator und eine Rückleitung für die Abführung der verbrauchten Dialysierflüssigkeit zur Auslaßleitung der Vorrichtung aufweist. Kurzschlußleitungen verbinden die Einlaßleitung der Trägerflüssigkeit mit der Auslaßleitung der verbrauchten Dialysierflüssigkeit sowie die Anschlußleitung der Mischeinrichtung zum Dialysator mit der Rückleitung der verbrauchten Dialysierflüssigkeit vom Dialysator. Wenn zunächst das Konzentratventil, das Bypassventil in der zweitgenannten Kurzschlußleitung und das Auslaßventil für die verbrauchte Dialysierflüssigkeit geschlossen werden und nach einer Zeitspanne das Einlaßventil für die Trägerflüssigkeit ebenfalls geschlossen wird, wird ein geschlossener Trägerflüssigkeitskreislauf gebildet, in dem aufgeheizte Trägerflüssigkeit mittels einer Pumpe umgewälzt wird, die das System vor dem Betrieb oder nach einer Betriebspause sterilisiert.

Der vorliegenden Erfindung liegt die Aufgabe zugrunde, ein Desinfektionsverfahren der betrachteten Art anzugeben, mit dem der gesamte Dialysierflüssigkeitskreislauf wirkungsvoll desinfiziert werden kann, so daß eine erneute Kontamination des Dialysegeräts bei Beginn der Dialyse zuverlässig vermieden ist. Außerdem soll der Energieverbrauch des Desinfektionsverfahrens gegenüber herkömmlichen Verfahren verringert sein.

Eine weitere Aufgabe der Erfindung besteht darin, ein Gerät zur Durchführung von Hygienmaßnahmen an einem Dialysegerät anzugeben, mit dem das Desinfektionsverfahren ausführbar ist. Zudem soll für den Anwendungsfall, daß das Desinfektionsverfahren eine chemische Desinfektion einschließt, eine Zuführeinrichtung zum dosierten Zuführen einer chemischen Desinfektionsflüssigkeit angegeben werden.

Diese Aufgaben werden erfindungsgemäß durch die im Kennzeichen der Patentansprüche 1, 3 und 6 angegebenen Merkmale gelöst. Vorteilhafte Weiterbildungen der Erfindung sind in den abhängigen Ansprüchen gekennzeichnet.

Bei dem erfindungsgemäßen Desinfektionsverfahren wird vor dem Zulaufhauptventil einer Frischwasserzulaufleitung, an dem die erste Stichleitung des Dialysegerätes angeschlossen wird, ein Zulaufvorventil angeordnet, wobei auf entsprechende Weise vor dem Wasserablaufhauptventil, an dem die zweite Stichleitung des Dialysegerätes angeschlossen wird, ein Ablaufvorventil angeordnet wird. Die zwischen Haupt- und Vorventil befindlichen Abschnitte der Wasserzulaufleitung und der Wasserablaufleitung werden mittels einer Querverbindungsleitung kurzgeschlossen, in der ein weiteres Ventil angeordnet wird. Zur Durchführung einer Desinfektionsmaßnahme werden zunächst die beiden Stichleitungen und das Dialysegerät mit Frischwasser durchgespült, indem alle Haupt- und Vorventile geöffnet werden, während hingegen das Querverbindungsventil geschlossen wird. Anschließend werden die Stichleitungen (mit den zugehörigen Verschraubungen und dergl.) sowie das Dialyesgerät in Rezirkulation einer Heißreinigung unterzogen, indem zuvor die Vorventile geschlossen werden, während das Querverbindungsventil geöffnet wird, wobei ferner die Heizquelle des Dialysegerätes für die Dauer der Heißreinigung eingeschaltet und die Pumpe des Dialysegerätes für die ausgewählte Zeitspanne in Betrieb gesetzt wird.

An den letzten Schritt des erfindungsgemäßen Desinfektionsverfahrens kann sich eine Dialysebehandlung anschließen, oder aber das Gerät wird abgeschaltet. Bevor dannzu einem späteren Zeitpunkt eine Dialysebehandlung ausgeführt wird, wird der Dialysekreislauf mit Frischwasser über eine ausgewählte Zeitspanne durchgespült.

Bei dem erfindungsgemäßen Verfahren werden alle Bestandteile des Dialysierflüssigkeitskreislaufes einer Desinfektion unterworfen, so daß weder von dem desinfizierten Dialysegerät noch von dessen Verteilungssystem eine Kontamination bei Beginn der Dialyse erfolgen kann. Da zudem der Heißreinigungsschritt in Rezirkulation erfolgt, ist hierbei der Energieverbrauch erheblich herabgesetzt, da das von dem Heizstab des Dialysegerätes erhitzte Wasser nach dem Durchlauf durch den Dialysierflüssigkeitskreislauf nicht sogleich abgeführt wird, sondern während des gesamten Heißreinigungsprozesses zirkuliert. Dabei werden außer dem Dialysegerät alle Schlauchstichleitungen mit ihren Verschraubungen und dergleichen wirkungsvoll desinfiziert.

Wenn das erfindungsgemäße Desinfektionsverfahren auch eine chemische Desinfektion einschließen soll, ist erfindungsgemäß vorgesehen, daß im Anschluß an das Durchspülen des Dialysierflüssigkreislaufes mit Frischwasser die Vorventile geschlossen werden, während das Querverbindungsventil geöffnet wird, daß eine Zuführeinrichtung, die ein bestimmte Menge eines flüssigen chemischen Desinfektionsmittels erhält, mit zwei zugehörigen Eingängen des Dialysegerätes verbunden wird, daß anschließend die Pumpe des Dialysegeräte über eine vorbestimmte Zeitspanne in Betrieb gesetzt wird, wodurch das chemische Desinfektionsmittel aus der Zuführeinrichtung in den Dialysierflüssigkeitskreislauf gebracht wird und in Rezirkulation ein chemischer Desinfektionsvorgang abläuft. Nach Beendigung dieses Vorgangs werden die Stichleitungen und das Dialysegerät von dem chemischen Desinfektionsmittel freigespült, nachdem die Pumpe abgestellte, das Querverbindungsventil geschlossen und die Vorventile geöffnet sind. Anschließend findet der bereits beschriebene Heißreinigungsvorgang statt.

Bei der chemischen Desinfektion werden demnach auch die beiden Stichleitungen und deren Zubehör wirkungsvoll desinfiziert. Da dieser Vorgang ebenfalls in Rezirkulation abläuft, ist hierbei eine weitaus geringere Dosierung von Desinfektionmitteln ausreichend, als dies bei herkömmlichen Verfahren der Fall ist, so daß auch aus diesem Grund das erfindungsgemäße Verfahren umweltfreundlicher und materialschonender ist.

Weitere Vorteile des erfindungsgemäßen Verfahrens liegen darin, daß die Dauer der Desinfektionsmaßnahmen wegen der durch die Rezirkulation erhöhten Effektivität gesenkt werden kann, so daß das Dialysegerät mehr Dialysebehandlungen pro Tag ausführen kann. Das erfindungsgemäße Desinfektionsverfahren trägt ferner zur Einsparung von Osmosewasser bei und schont die Bauteile des Dialysierflüssigkeitkreislaufes, so daß deren Bestandteile eine längere Nutzungsdauer haben.

Das erfindungsgemäße Gerät zur Durchführung von Hygienemaßnahmen, insbesondere zur Durchführung des erfindungsgemäßen Desinfektionsverfahrens, enthält in einem Gehäuse einen Frischwasserzulaufleitungsabschnitt mit einem Anschluß für eine gebäudeseitige Frischwasserzulaufleitung und einem Vorventil, dem ein Zwischenleitungsabschnitt, ein Hauptventil und ein Anschluß für eine Zulaufstichleitung des Dialysegerätes folgen. In entsprechender Weise ist ein Wasserablaufleitungsabschnitt mit einem Anschluß für eine gebäudeseitige Wasserablaufleitung und einem Vorventil vorgesehen, dem ein Zwischenleitungsabschnitt, ein Hauptventil und ein Anschluß für eine Ablaufstichleitung des Dialysegerätes folgen. Die Zwischenleitungsabschnitte sind durch einen Querverbindungsleitungsabschnitt verbunden, der mit einem Querverbindungsventil versehen ist. Außerdem ist eine Ventilschaltung angeordnet, die für jedes der Ventile eine Ansteuerungsleitung aufweist und über einen Geräteanschluß elektrisch mit dem Dialysegerät verbindbar ist, so daß die Ventile nach vorgegebenen Programmen des Dialysegerätes schaltbar sind.

Mit dem Zusatzgerät ist demnach an allen Typen von Dialysegeräten das erfindungsgemäße Desinfektionsverfahren ausführbar, bei dem die Hauptventile, Vorventile und das Querverbindungsventil entsprechend dem Programm des Dialysegerätes geschaltet werden.

Nach einem weiteren Vorschlag der Erfindung enthält das erfindungsgemäße Gerät eine Einschaltuhr, mit der der Start eines Hygiene-Programms einstellbar ist, so daß hierzu nicht die Anwesenheit einer Bedienungsperson erforderlich ist.

Mit besonderem Vorteil wird eine Fail-safe-Schaltung für die Ventile vorgeschlagen, die so wirkt, daß die einzelnen Schritte eines Hygienprogramms bzw. Desinfektionsprogramms nur dann gestartet werden, wenn sich alle Ventile in der korrekten Stellung befinden. Hierzu können die Ventile als Rückmelder mit einem kleinen Endschalter versehen werden, dessen jeweilige Position abgefragt wird, so daß beispielsweise ein Spülprogramm erst dann gestartet wird, wenn sichergestellt ist, daß sich alle Ventile in der korrekten Stellung befinden.

Das erfindungsgemäße Gerät enthält ferner eine schematische Anzeige der Ventile, die beispielsweise durch Lichtzeichen anzeigt, welche Ventile geöffnet bzw. geschlossen sind.

Nach einem weiteren Vorschlag der Erfindung können die Vorventile und das Querverbindungsventil mit ihren Leitungsabschnitten auch Teil einer gebäudeseitigen Versorgungskonsole bzw. Versorgungsschiene sein. Hierzu wird eine erfindungsgemäße Wasserversorgungseinrichtung vorgeschlagen, bei der vor dem bisher angeordneten einzigen Zulaufventil ein Zulaufvorventil in der Zulaufleitung und vor dem Ablaufventil ein Ablaufvorventil in der Ablaufleitung (jeweils an der von dem Dialysegerät abgewandten Seite) angeordnet sind, und die beiden Leitungsabschnitte zwischen den in Reihe angeordneten Ventilen mit einer Querverbindungsleitung verbunden sind, die ein Querverbindungsventil aufweist. Hierbei sollen wiederum alle Ventile von einer automatischen Betätigungseinrichtung, vorzugsweise elektromagnetisch , betätigbar sein, so daß sie von dem Programm eines angeschlossenen Dialysegerätes geschaltet werden können, wobei selbstverständlich entsprechende elektrische Schaltungen und Verbindungen vorgesehen sind.

Die erfindungsgemäße Zuführeinrichtung zum Zuführen zweier Flüssigkeiten enthält einen Hohlzylinder, an dessen Innenwand ein verschieblicher Kolben flüssigkeitsdicht anliegt, der eine vordere und eine rückwärtige Zylinderkammer begrenzt, deren Volumen durch die Bewegung des Kolbens veränderbar ist. Im rückwärtigen Endbereich hat der Hohlzylinder eine Flüssigkeits-Eintrittsöffnung, durchdie bei dem erfindungsgemäßen Desinfektionsverfahren Wasser eintritt, und im vorderen Endbereich eine Öffnung, durch die eine Flüssigkeit in die vordere Zylinderkammer einfüllbar ist, wobei dies bei dem erfindungsgemäßen Desinfektionsverfahren das chemische Desinfektionmittel ist. Die vordere Öffnung dient ferner dem Austritt beider Flüssigkeiten, wobei zudem eine Flüssigkeit-Durchlaßeinrichtung von der hinteren zur vorderen Zylinderkammer vorgesehen ist, die in einer vorgegebenen, vorzugsweise vorgeschobenen Position des Kolbens den Austritt der in der rückwärtigen Zylinderkammer befindlichen Flüssigkeit in die vordere Zylinderkammer und aus der vorderen Öffnung des Hohlzylinders heraus zuläßt.

Bei dem erfindungsgemäßen Verfahren wird vorzugsweise mittels einer automatischen Füllvorrichtung unter Druck ein chemisches Desinfektionsmittel in einer vorbestimmten Menge durch die vordere Öffnung in die vordere Zylinderkammer eingefüllt, wobei die Öffnung durch ein Ventil schließbar sein kann, das unter Druck in beiden Richtungen öffnen kann.

Nach einem weiteren Vorschlag der Erfindung sind an der Zuführeinrichtung vorzugsweise seitlich zwei Konnektoren angebracht, mit denen die Zuführeinrichtung an dem Dialysegerät befestigt werden kann, wobei durch einen der Konnektoren die Flüssigkeit (Wasser) in die hintere Zylinderkammer eingeführt wird. Erfindungsgemäß ist ferner vorgesehen, daß durch den Eingriff der Konnektoren ein elektrischer Kontakt hergestellt wird, der die automatische Einstellung eines Desinfektionsprogramms unter Einschluß einer chemischen Desinfektion bewirkt.

Die Flüssigkeit-Durchlaßeinrichtung zwischen der rückwärtigen und der vorderen Zylinderkammer ist vorzugsweise durch mehrere axiale Bohrungen gebildet, die von außen durch den vorderen Zylinderboden hindurch Umfangsabschnitte der Innenwand des inneren vorderen Endabschnitts des Hohlzylinders wegschneiden. Anschließend wird der Zylinderboden dadurch geschlossen, daß zylindrische Füllstücke eingesetzt, vorzugsweise eingeklebt werden, die den vorderen Zylinderboden wieder schließen. Damit verbleiben lediglich im vorderen Endabschnitt der Zylinderinnenwand Aufweitungen, an denen die in der hinteren Zylinderkammer befindliche, unter Druck stehende Flüssigkeit den vorzugsweise durch einen O-Ring seitlich abgedichteten Kolben umströmen kann.

Damit die vordere Zylinderkammer der Zuführeinrichtung ohne zusätzliche Handgriffe stets wieder mit einer Flüssigkeit befüllt werden kann, wird erfindungsgemäß vorgeschlagen, daß zwischen dem vorderen Hohlzylinderboden und dem Kolben eine Federeinrichtung angeordnet ist, vorzugsweise in Form einer in axialer Richtung verlaufenden Schraubenfeder, die komprimiert wird, wenn der Kolben in den Bereich der Flüssigkeitdurchlaßeinrichtung vorgeschoben wird, so daß die Federkraft den Kolben wieder in einen rückwärtigen Bereich zurückdrückt, indem dieser dicht an der Zylinderinnenwand anliegt, wenn der Kolben nicht mehr durch Flüssigkeitsdruck in Vorschubrichtung beaufschlagt ist. Damit wird nach Beendigung eines chemischen Desinfektionsschritts des erfindungsgemäßen Desinfektionsverfahrens der Kolben automatisch in eine Position zurückgedrückt, in der die beiden Zylinderkammern voneinander getrennt sind, so daß die vordere Kammer wieder mit einer Flüssigkeit befüllt werden kann. Dies kann selbstverständlich nicht nur durch eine automatische Füllvorrichtung, sondern auch von Hand geschehen.

Weitere Einzelheiten der Erfindung ergeben sich aus der nachfolgenden Beschreibung einiger bevorzugter Ausführungsformen sowie anhand der Zeichnung. Dabei zeigen:
- Fig. 1: ein Ablaufdiagramm einer Heißreinigung eines Dialysierflüssigkeitskreislaufes gemäß der Erfindung;
- Fig. 2: ein Ablaufdiagramm eines Desinfektionsverfahrens, bei dem eine chemische Desinfektion mit einer Heißreinigung kombiniert ist,gemäß der Erfindung;
- Fig. 3: ein Gerät zur Durchführung von Hygienemaßnahmen an einem Dialysegerät;
- Fig. 4: einen Längsschnitt durch eine Zuführeinrichtung;
- Fig. 5: den vorderen Zylinderboden der Zuführeinrichtung gemäß Fig. 4 nach einem ersten Arbeitsschritt zur Herstellung der Flüssigkeit-Durchlaßeinrichtung;
- Fig. 6: den Bereich des Kolbens der Zuführeinrichtung in einer vergrößerten Darstellung.

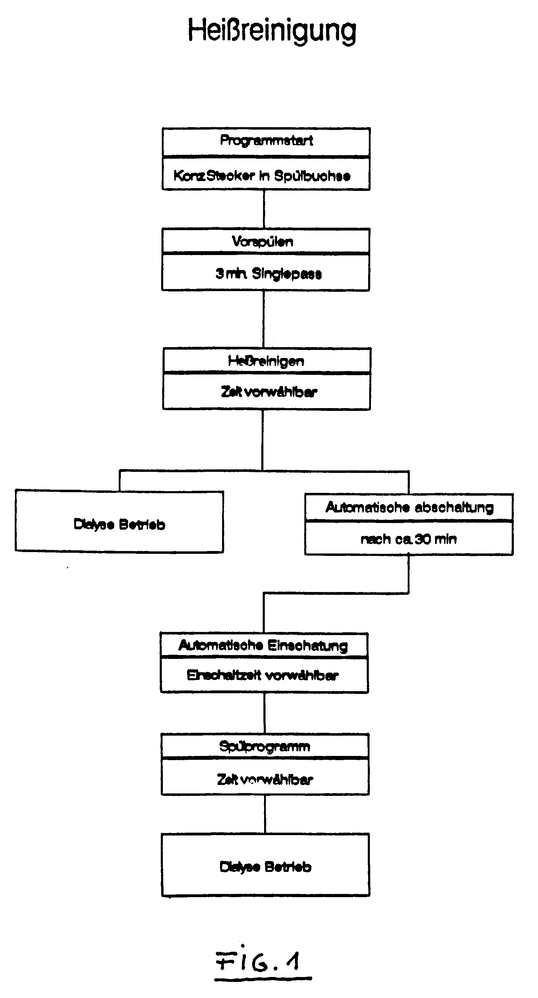

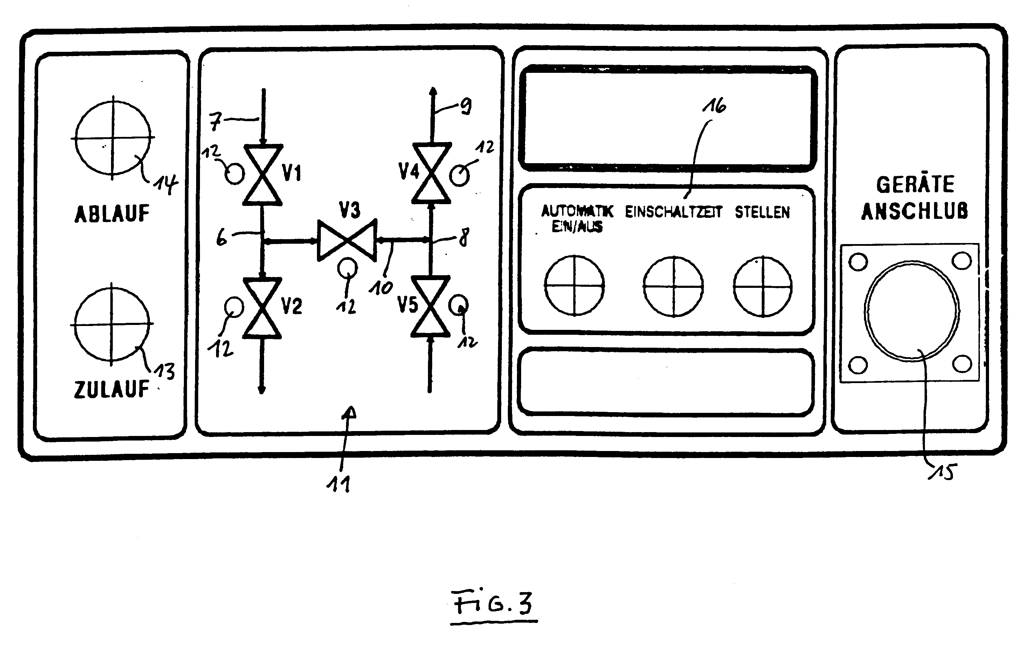

In Figur 1 ist ein Ablaufdiagramm einer Heißreinigung dargestellt, bei der der erste Schritt der Frischwasserspülung des Dialysierflüssigkeitskreislaufs drei Minuten lang dauert, während die Zeitspanne des anschließenden Heißreinigungsschrittes vorwählbar ist. Wenn im Anschluß an die Heißreinigung nicht direkt ein Dialysebetrieb folgt, kann nach der automatischen Abschaltung das nachfolgende Spülprogramm mit der Einschaltuhr des in Figur 3 abgebildeten Gerätes vorgewählt werden. Die Zeitspanne dieses Spülprogramms ist vorwählbar.

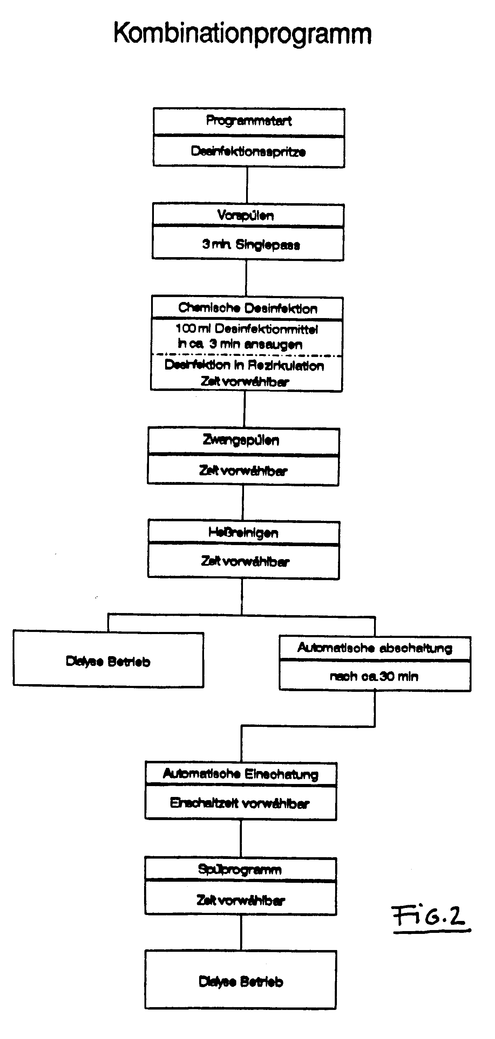

Auch bei dem in Figur 2 abgebildeten Kombinationsprogramm findet zunächst ein 3-minütiges Freispülen des Dialysegerätes mit dem zugehörigen Verteilungssystem statt, woran sich der chemische Desinfektionsschritt anschließt, dessen Zeitdauer vorwählbar ist. Hierzu wird die Zuführeinrichtung mit 100 ml Desinfektionsmittel gefüllt, das innerhalb von drei Minuten in den Dialysierflüssigkeitskreislauf eingespeist wird. Im übrigen ergeben sich die einzelnen Verfahrensschritte aus dem Ablaufdiagramm.

Das in Figur 3 abgebildete Gerät zur Durchführung von Hygienemaßnahmen an einem Dialysegerät enthält eine schematische Darstellung der Ventilanordnung des Gerätes, die aus einem Vorventil V1, einem Zwischenleitungsabschnitt 6 und einem Hauptventil V2 der Frischwasserzulaufleitung 7, einem Vorventil V4, einem Zwischenleitungsabschnitt 8 und einem Hauptventil V5 der Wasserablaufleitung 9 sowie einem Querverbindungsventil V3 in einem Querverbindungsleitungsabschnitt 10 besteht.

Die schematische Abbildung 11 dieser in dem Gerät befindlichen Ventilanordnung ist bei jedem Ventil mit einem Signallämpchen 12 versehen, das den jeweiligen Schaltzustand des Ventils anzeigt.

Wie erwähnt, sind die Vorventile V1 und V4 mit einer gebäudeseitigen Frischwasserzulaufleitung und einer gebäudeseitigen Wasserablaufleitung verbunden. Zum Anschluß des Dialysegerätes an diese Wasserleitungen ist in dem Zusatzgerät der Zulauf 13 sowie der Ablauf 14 vorgesehen.

Das Gerät hat ferner einen Geräteanschluß 15 in Form eines Buchsensteckers, um das Dialysegerät mit der in dem Zusatzgerät befindlichen Ventilschaltung kurzzuschließen, damit die Ventile V1 bis V5 entsprechend dem Programm des Dialysegerätes geschaltet werden können. Außerdem enthält das in Figur 3 abgebildete Zusatzgerät eine Einschaltuhr 16, mit der die Einschaltzeit der gewünschten Programme vorwählbar ist.

Die in Figur 4 abgebildete Zuführeinrichtung enthält einen Hohlzylinder 17, in dem ein Kolben 18 hin- und herbewegbar ist, der den Innenraum des Hohlzylinders 17 in einen hinteren und einen vorderen Zylinderraum 19, 20 unterteilt. Der Kolben 18 ist mittels eines O-Ringes 21 aus Silikon gegenüber der Innenwand des Zylinders 17 abgedichtet.

Der in der Figur obere Zylinderboden 22 ist mit der rohrförmigen Wand des Zylinders 17 verklebt und enthält die obere bzw. rückwärtige Eintrittsöffnung 23 für eine erste Flüssigkeit (Wasser),die durch einen eingesteckten Konnektor 24 zugeführt wird, mit dem die Zuführeinrichtung an einem Dialysegerät befestigbar ist. Hierzu ist ferner am anderen axialen Ende ein weiterer seitlicher Konnektor 25 vorgesehen, der nur der Befestigung, nicht jedoch einer Flüssigkeitszufuhr dient. Außerdem stellen die beiden Konnektoren 24, 25 einen elektrischen Kontakt her, mit dem dem Dialysegerät signalisiert wird, daß ein chemischer Desinfektionsvorgang ausführt werden soll.

In dem unteren (vorderen) Zylinderboden 26 befindet sich eine zentrale vordere Öffnung 27, durch die eine zweite Flüssigkeit, z.B. ein flüssiges chemisches Desinfektionsmittel, in die vordere Zylinderkammer 20 eingeführt werden kann. Die Öffnung 27 ist mittels eines Ventils verschlossen, das unter Druck in beiden Richtungen öffnen kann. Die vordere Öffnung 27 dient ferner dem Austritt der in der vorderen Zylinderkammer 20 befindlichen ersten Flüssigkeit sowie dem Austritt der Flüssigkeit der hinteren Zylinderkammer 19, wenn diese den Kolben 18 umströmen kann.

Hierzu ist in dem vorderen (unteren) Endbereich der Innenwand des Hohlzylinders 17 eine Flüssigkeitdurchlaßeinrichtung in Form von mehreren kreisförmigen Bohrungen 28 vorgesehen. Wenn der Kolben 18 soweit vorgeschoben ist, daß sich sein Dichtring 21 im Bereich der durch die Bohrungen 28 geschaffenen Aufweitung befindet, kann die in der hinteren Zylinderkammer 19 befindliche Flüssigkeit den Dichtring 21 radial außen umströmen und gelangt in die vordere Zylinderkammer 20, aus der sie die restliche Flüssigkeitsmenge (Desinfektionsmittel) ausführt und selbst aus der Öffnung 27 austritt.

Aus Figur 5 ist zu ersehen, daß die Flüssigkeitsdurchlaßeinrichtung dadurch ausgebildet wird, daß von der unteren Stirnseite her mehrere zylindrische Bohrungen ausgeführt werden, die anschließend im Bereich des Zylinderbodens 26 wieder durch entsprechend geformte Füllstücke geschlossen werden, die beispielsweise eingeklebt werden. Danach verbleiben noch die in Figur 4 abgebildeten Aussparungen in der Zylinderinnenwand.

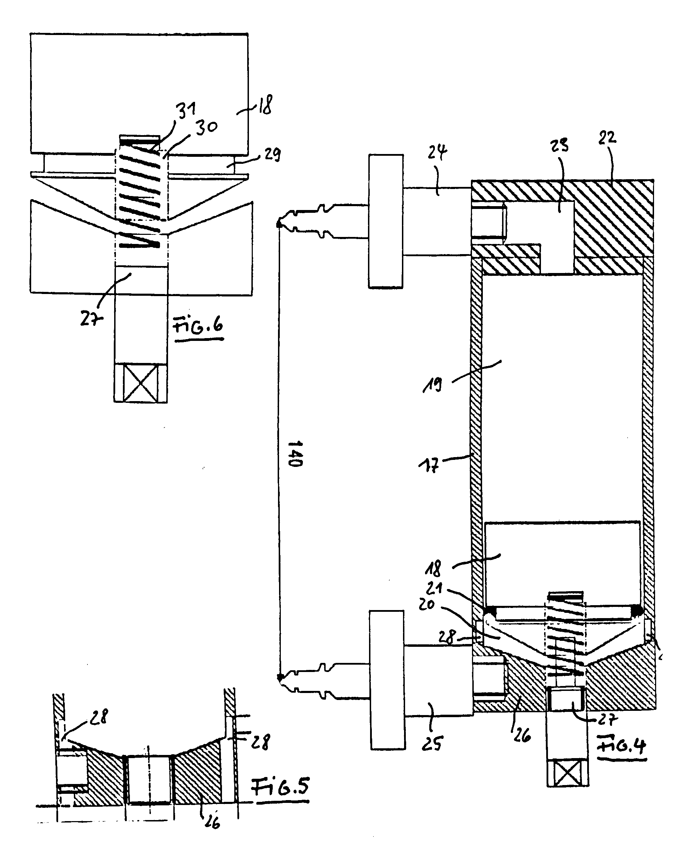

Der in Figur 6 vergrößert dargestellte Kolben 18 enthält eine Ringnut 29 zur Aufnahme des O-Ringes 21 und eine Bohrung 30 zur Aufnahme der Schraubenfeder 31, deren Gegensitz durch das Ventil der Öffnung 27 gebildet ist. Wenn der Kolben eine derart vorgeschobene Position erreicht hat, daß sich sein O-Ring 21 im Bereich der Aufweitungen 28 befindet, ist die Schraubenfeder 31 so weit zusammengedrückt, daß sie den Kolben 18 selbsttätig wieder aus dem Bereich der Aufweitungen 28 zurückdrückt, wenn dieser nicht mehr von einem Flüssigkeitsdruck beaufschlagt ist. Damit ist wieder eine flüssigkeitsdichte Trennung zwischen der hinteren und der vorderen Zylinderkammer 19, 20 geschaffen, so daß die vordere Kammer 20 wieder mit einem Desinfektionsmittel gefüllt werden kann, ohne daß dieses zur hinteren Kammer 19 strömt.

Wie die Figuren 4 und 6 zeigen, hat der Kolben an seiner (unteren) Vorderseite die Form eines Kegelstumpfes, während der Zylinderboden 26 an seiner Innenseite eine negative Kegelstumpfform hat. Dabei ist der Neigungswinkel des Kolbens größer als derjenige des Zylinderbodens. Durch diese Formgebung ist das glatte Ausführen der in der vorderen Zylinderkammer 20 befindlichen Flüssigkeit sowie das Ausströmen der Flüssigkeit aus der hinteren Zylinderkammer erleichtert.

Das Zuführgerät ist nicht nur zur chemischen Desinfektion eines Dialysegerätes geeignet, sondern allgemein zum Zuführen von flüssigen oder gasförmigen Medien, wobei der Kolben nicht nur durch Überdruck in der rückwärtigen Zylinderkammer, sondern auch durch Unterdruck in der vorderen Zylinderkammer (Sog) vorgeschoben werden kann.

## Patentansprüche

1. Desinfektionsverfahren eines Dialysierflüssigkeitskreislaufes, der eine Dialysemaschine mit einer ersten Stichleitung, die an ein Zulaufhauptventil (V2) einer Frischwasserzulaufleitung (7) angeschlossen wird, und eine zweite Stichleitung, die an ein Ablaufhauptventil (V5) einer Wasserablaufleitung (9) angeschlossen wird, enthält, wobei dem Zulaufhauptventil (V2) ein Zulaufvorventil (V1) und dem Ablaufhauptventil (V5) ein Ablaufvorventil (V4) jeweils an der von dem Dialysegerät abgewandten Seite vorgeschaltet ist und eine mit einem Querverbindungsventil (V3) versehene Querverbindungsleitung (10) die Leitungsabschnitte zwischen den Hauptventilen (V2, V5) und den Vorventilen (V1, V4) verbindet,
wobei das Verfahren folgende Schritte aufweist:
a) die beiden Stichleitungen und das Dialysegerät werden mit Frischwasser durchgespült, indem alle Hauptventile (V2, V5) und Vorventile (V1, V4) geöffnet werden und das Querverbindungsventil (V3) geschlossen wird;
b) die Stichleitungen und das Dialysegerät werden einer Heißreinigung in Rezirkulation unterzogen, indem die Vorventile (V1, V4) geschlossen werden und das Querverbindungsventil (V3) geöffnet wird, die Heizquelle des Dialysegerätes eingeschaltet wird und die Pumpe des Dialysegerätes eine ausgewählte Zeitspanne lang in Betrieb gesetzt wird.

2. Desinfektionsverfahren nach Anspruch 1,
ferner gekennzeichnet durch folgende Schritte im Anschluß an Schritt a):
aa) die Vorventile (V1, V4) werden geschlossen, und das Querverbindungsventil (V3) wird geöffnet;
ab) eine Zuführeinrichtung mit einem flüssigen chemischen Desinfektionsmittel wird mit dem Konzentrateingang des Dialysegerätes und mit einer Wasserzulaufleitung des Dialysegerätes verbunden;
ac) die Pumpe des Dialysegerätes wird in Betrieb gesetzt, wodurch das chemische Desinfektionsmittel aus der Zuführeinrichtung in den Dialysierflüssigkeitskreislauf gerät und in Rezirkulation ein chemischer Desinfektionsvorgang ausgeführt wird, und
ad) die Stichleitungen und das Dialysegerät werden von dem chemischen Desinfektionsmittel freigespült, nachdem die Pumpe abgestellt, das Querverbindungsventil (V3) geschlossen und die Vorventile (V1, V4) geöffnet sind.

3. Gerät zur Durchführung von Hygienemaßnahmen an einem Dialysegerät,
gekennzeichnet durch einen Frischwasserzulaufleitungsabschnitt (7) mit einem Anschluß für eine Frischwasserzulaufleitung und einem Vorventil (V1), dem ein Zwischenleitungsabschnitt (6), ein Hauptventil (V2) und ein Anschluß (13) für eine Zulaufstichleitung des Dialysegerätes folgen, einen Wasserablaufleitungsabschnitt (9) mit einem Anschluß für eine Wasserablaufleitung und ein Vorventil (V4), dem ein Zwischenleitungsabschnitt (8), ein Hauptventil (V5) und ein Anschluß (14) für eine Ablaufstichleitung des Dialysegerätes folgen, einen die Zwischenleitungsabschnitte (6, 8) verbindenden Querverbindungsleitungsabschnitt (10) mit einem Querverbindungsventil (V3), und mit einer Ventilschaltung, die für jedes Ventil eine Ansteuerungsleitung aufweist und über einen Geräteanschluß (15) elektrisch mit dem Dialysegerät verbindbar ist, so daß die Ventile nach vorgegebenen Programmen des Dialysegerätes schaltbar sind.

4. Gerät nach Anspruch 3,
ferner gekennzeichnet durch eine Einschaltuhr (16), mit der der Start eines Hygieneprogramms frei einstellbar ist.

5. Gerät nach Anspruch 3 oder 4,
ferner gekennzeichnet durch eine Fail-safe-Schaltung der Ventile, die so wirkt, daß die einzelnen Schritte eines Hygieneprogramms nur dann gestartet werden, wenn sich alle Ventile (V1-V5) in der korrekten Stellung befinden.

6. Wasserversorgungseinrichtung für ein Dialysergerät, mit einer Frischwasserzulaufleitung mit einem Zulaufventil (V2), an das eine erste Stichleitung des Dialysegerätes anschließbar ist, und mit einer Wasserablaufleitung mit einem Ablaufventil (V5), an das eine zweite Stichleitung des Dialysegerätes anschließbar ist,
dadurch gekennzeichnet, daß vor dem Zulaufventil (V2) ein Zulaufvorventil (V1) in der Zulaufleitung (7) und vor dem Ablaufventil (V5) ein Ablaufvorventil (V4) in der Ablaufleitung (9), jeweils an der von dem Dialysegerät abgewandten Seite, angeordnet sind, und daß die beiden Leitungsabschnitte (6, 8) zwischen den hintereinander angeordneten Ventilen (V1, V2; V4, V5) mit einer Querverbindungsleitung (10) verbunden sind, die ein Querverbindungsventil (V3) aufweist.

7. Wasserversorgungseinrichtung nach Anspruch 6,
dadurch gekennzeichnet, daß alle Ventile (V1-V5) von einer automatischen Betätigungseinrichtung, vorzugsweise elektromagnetisch, betätigbar sind.

## Claims

1. A method of disinfecting a dialysis fluid circuit consisting of a dialysis machine including a first connection conduit connectable to a main inlet-valve (V2) of a fresh water inlet conduit (7), and a second connection conduit connectable to a main outlet-valve (V5) of a water outlet conduit (9), said main inlet-valve (V2) having arranged upstream thereof an inlet prevalve (V1) and said main outlet-valve (V5) an outlet prevalve (V4), each at the side facing away from the dialysis apparatus, and a transverse connection conduit (10), which is provided with a transverse connection valve (V3) connecting the conduit portions between said main valves (V2, V5) and prevalves (V1, V4) said method comprising the following steps:
a) flushing said two connection conduits and said dialysis apparatus with fresh water by opening all main valves (V2, V5) and prevalves (V1, V4) and by closing said transverse connection valve (V3);
b) subjecting said connection conduits and said dialysis apparatus to hot cleaning in a recirculatory process by closing said prevalves (V1, V4) and opening said transverse connection valve (V3), switching on the heating source of said dialysis apparatus and operating the pump of said dialysis apparatus for a preselected period of time.

2. A disinfecting method as defined in claim 1,
further characterized by the following steps which are taken after step a):
aa) closing said prevalves (V1, V4) and opening said transverse conncection valve (V3);
ab) connecting a supply means containing a liquid chemical disinfectant to the concentrate inlet of said dialysis apparatus and to a water inlet conduit of said dialysis apparatus;
ac) putting said pump of said dialysis apparatus into operation, whereby said chemical disinfectant passes from said supply means into said dialysis fluid circuit and a chemical disinfection operation is carried out in a recirculatory process; and
ad) flushing said connection conduits and said dialysis apparatus to free them from said chemical disinfectant after said pump has been put out of operation, said transverse connection valve (V3) has been closed and said prevalves (V1, V4) have been opened.

3. An apparatus for carrying out hygienic operations on a dialysis apparatus, characterized by a fresh water inlet conduit portion (7) comprising a connection for a fresh water inlet conduit and a prevalve (V1) followed by an intermediate conduit portion (6), a main valve (V2) and a connection (13) for an inlet connection conduit of said dialysis apparatus, by a water outlet conduit portion (9) comprising a connection for a water outlet conduit and a prevalve (V4) followed by an intermediate conduit portion (8), a main valve (V5) and a connection (14) for an outlet connection conduit of said dialysis apparatus, by a transverse connection conduit portion (10) connecting said intermediate conduite portions (6, 8) and comprising a transverse connection valve (V3), and a valve switching means including a control conduit for each of said valves and being electrically connectable to said dialysis apparatus via an apparatus coupling (15), so that said valves are switchable in accordance with given programs of said sialysis apparatus.

4. An apparatus as defined in claim 3,
further characterized by a switch-on clock (16) with which the start of a hygiene program is freely adjustable.

5. An apparatus as defined in claim 3 or 4,
further characterized by a fail-safe circuit of said valves which acts such that the individual steps of a hygiene program can only be taken if all of said valves (V1-V5) are in their correct positions.

6. A water supply means for a dialysis apparatus comprising a fresh water inlet conduit including an inlet valve (V2) to which a first connection conduit of said dialysis apparatus is connectable, and a water outlet conduit including an outlet valve (V5) to which a second connection conduit of said dialysis appartaus is connectable,
characterized in that an inlet prevalve (V1) is arranged in front of said inlet valve (V2) in said inlet conduit (7) and an outlet prevalve (V4) is arranged in front of said outlet valve (V5) in said outlet conduit (9), both of said prevalves being disposed at the side facing away from said dialysis appartus, and that the two conduit portions (, 6, 8) which are provided between said vaves (V1, V2; V4, V5) arranged one after the other are connected to a transverse connection conduit (10) comprising a transverse connection valve (V3).

7. A water supply means as defined in claim 6,
characterized in that all of said valves (V1-V5) are actuable by an automatic actuation means, preferably in an electromagnetic way.

## Revendications

1. Procédé de désinfection d'un circuit de liquide de dialyse, qui contient une machine de dialyse ayant une première conduite de branchement, qui est raccordée à une vanne principale d'arrivée (V2) d'une conduite d'arrivée d'eau fraîche (7), et une deuxième conduite de branchement, qui est raccordée à une vanne d'évacuation principale (V5) d'une conduite d'évacuation d'eau (9), où, chaque fois, au coté éloigné de l'appareil de dialyse, une vanne avant d'arrivée (V1) est mise en circuit avant la vanne principale d'arrivée (V2) et une vanne avant d'évacuation (V4) est mise en circuit avant la vanne principale d'évacuation (V5), et une conduite de liaison transversale (10) pourvue d'une vanne de liaison transversale (V3) relie les sections de conduite entre les vannes principales (V2, V5) et les vannes avant (V1, V4),
où le procédé présente les étapes suivantes :
a) les deux conduites de branchement et l'appareil de dialyse sont rincés avec de l'eau fraîche, tandis que toutes les vannes principales (V2, V5) et toutes les vannes avant (V1, V4) sont ouvertes et la vanne de liaison transversale (V3) est fermée ;
b) les conduites de branchement et l'appareil de dialyse sont soumis à un nettoyage à chaud en recirculation, tandis que les vannes avant (V1, V4) sont fermées et la vanne de liaison transversale (V3) est ouverte, la source chauffante de l'appareil de dialyse est en service et la pompe de l'appareil de dialyse est mise en exploitation pendant un intervalle de temps sélectionné.

2. Procédé de désinfection selon la revendication 1, caractérisé de plus par des étapes suivantes en liaison avec l'étape a) :
aa) les vannes avant (V1, V4) sont fermées, et la vanne de liaison transversale (V3) est ouverte ;
ab) un agencement d'amenée, ayant un désinfectant chimique liquide, est relié avec l'entrée de concentré de l'appareil de dialyse et avec une conduite d'arrivée d'eau de l'appareil de dialyse ;
ac) la pompe de l'appareil de dialyse est mise en exploitation, grâce à quoi le désinfectant chimique s'engage, à partir de l'agencement d'amenée, dans le circuit de liquide de dialyse et un processus de désinfection chimique est exécuté en recirculation, et
ad) les conduites de branchement et l'appareil de dialyse sont libérées par rinçage du désinfectant chimique, après quoi la pompe est arrêtée, la vanne de liaison transversale (V3) est fermée et les vannes avant (V1, V4) sont ouvertes.

3. Appareil pour le mise en oeuvre de mesures d'hygiène dans un appareil de dialyse, caractérisé par une section de conduite d'arrivée d'eau fraîche (7) ayant un raccordement pour une conduite d'arrivée d'eau fraîche et une vanne avant (V1), que suivent une section de conduite intermédiaire (6), une vanne principale (V2) et un raccordement (13) pour une conduite de branchement d'arrivée de l'appareil de dialyse, une section de conduite d'évacuation d'eau (9) ayant un raccordement pour une conduite d'évacuation d'eau et une vanne avant (V4), que suivent une section de conduite intermédiaire (8), une vanne principale (V5) et un raccordement (14) pour une conduite de branchement d'évacuation de l'appareil de dialyse, une section de conduite de liaison transversale (10) ayant une vanne de liaison transversale (V3) reliant les sections de conduite intermédiaires (6, 8), et ayant un circuit de vannes, qui, pour chaque vanne, présente une ligne électrique de commande et est reliable, par l'intermédiaire d'un raccordement d'appareil (15), électriquement avec l'appareil de dialyse, de sorte que les vannes sont commutables selon des programmes donnés par avance de l'appareil de dialyse.

4. Appareil selon la revendication 3, caractérisé de plus par une pendule de mise en service (16), avec laquelle le démarrage d'un programme d'hygiène est ajustable librement.

5. Appareil selon la revendication 3 ou la revendication 4, caractérisé de plus par un circuit de sécurité en cas de défaillance des vannes, qui agit ainsi, que les étapes individuelles d'un programme d'hygiène sont seulement alors démarrées lorsque toutes les vannes (V1 à V5) se trouvent dans la position correcte.

6. Agencement de fourniture d'eau pour un appareil de dialyse, ayant une conduite d'arrivée d'eau fraîche avec une vanne d'arrivée (V2), à laquelle est raccordable une première conduite de branchement de l'appareil de dialyse, et ayant une conduite d'évacuation d'eau avec une vanne d'évacuation (V5), à laquelle est raccordable une deuxième conduite de branchement de l'appareil de dialyse,
caractérisé en ce que, chaque fois, au côté éloigné de l'appareil de dialyse, avant la vanne d'amenée (V2) est agencée une vanne avant d'amenée (V1) dans la conduite d'amenée (7) et avant la vanne d'évacuation (V5) est agencée une vanne avant d'évacuation (V4) dans la conduite d'évacuation (9), et que les deux sections de conduite (6, 8), entre les vannes agencées l'une derrière l'autre (V1, V2 ; V4, V5), sont reliées avec une conduite de liaison transversale (10), qui présente une vanne de liaison transversale (V3).

7. Agencement de fourniture d'eau selon la revendication 6, caractérisé en ce que toutes les vannes (V1 à V5) sont actionnables par un agencement d'actionnement automatique, de façon préférée électromagnétique.
